Europäisches Patentamt

European Patent Office

Office européen des brevets

(11) Veröffentlichungsnummer: **0 278 303**
**A1**

(12) **EUROPÄISCHE PATENTANMELDUNG**

(21) Anmeldenummer: 88101037.5

(22) Anmeldetag: 25.01.88

(51) Int. Cl.⁴: **A61B 17/22**

(30) Priorität: 04.02.87 DE 3703332
04.02.87 DE 3703333

(43) Veröffentlichungstag der Anmeldung:
17.08.88 Patentblatt 88/33

(84) Benannte Vertragsstaaten:
**DE FR GB NL**

(71) Anmelder: **Siemens Aktiengesellschaft Berlin
und München**
**Wittelsbacherplatz 2**
**D-8000 München 2(DE)**

(72) Erfinder: **Hassler, Dietrich**
**Flurweg 3**
**D-8525 Uttenreuth(DE)**
Erfinder: **Schmidt, Erhard**
**Heuwaagstrasse 20A**
**D-8520 Erlangen(DE)**

(54) Lithotripter mit integriertem Sektorscanner.

(57) Der Lithotripter zur Zertrümmerung eines Konkrements (19) im Körper eines Patienten (17) umfaßt eine Stoßwellenimpulse aussendende Stoßwellenquelle (1) und eine Fokussierungseinrichtung (11). In Ausbreitungsrichtung der Stoßwellenimpulse gesehen hinter oder vor der Fokussierungseinrichtung (11) ist das dreh- oder schwenkbare Element (21; 27) eines mechanischen Sektorscanners angeordnet. Die Drehachse (23) dieses Elements (21; 27) steht senkrecht auf der Zentralachse (25) der Fokussierungseinrichtung (11). Eine Synchronisiereinrichtung sorgt dafür, daß nur bei Seitwärtsstellung des Elements (21; 27) Stoßwellenimpulse ausgelöst werden, so daß eine Stoßwellen-Abschattung durch das Element (21; 27) selbst weitgehend vermieden wird. Vorteil des Lithotripters ist es, daß während der gesamten Lithotripsiebehandlung des Patienten (17) die Lage des Konkrements (19) beobachtet werden kann. Wenn sich das Konkrement (19) aus der Fokuszone (F) verlagert, wird dies im Ultraschall-Bild erkannt, und es kann eine Nachführung des Fokus (F) vorgenommen werden. Ist das Element (21; 27) vor der Fokussierungseinrichtung (11) angeordnet, ergibt sich der Vorteil, daß das vom Fokus (F') sektorförmig ausgesandte Ultraschall-Ortungssignal (31) durch die Fokussierungseinrichtung (11) in einen Parallelscan umgeformt wird, so daß der Weg des Stoßwellenimpulses vollständig überwacht werden kann, z.B. auf Ultraschall-Hindernisse.

FIG 1

## Lithotripter mit integriertem Sektorscanner

Die Erfindung betrifft einen Lithotripter zur Zertrümmerung eines Konkrementes im menschlichen Körper mit einem Stoßwellenrohr, das an seinem einen Ende mit einem Stoßwellenimpulse aussendenden Stoßwellenerzeuger und an seinem anderen Ende mit einer Ankoppelmembran abgeschlossen ist und mit einer Fokussierungseinrichtung im Stoßwellenrohr.

Eine solche Vorrichtung ist beispielsweise aus der deutschen Offenlegungsschrift 33 28 039 bekannt. Dort ist als Stoßwellen-quelle ein Stoßwellenrohr eingesetzt, welches eine elektrische Spule, eine Isolierfolie und eine Kupfermembran umfaßt. Wird auf die Spule ein Stromimpuls gegeben, so werden in der vorgelagerten Membran Wirbelströme erzeugt, die die Membran von der Spule wegschlagen. In dem angrenzenden Übertragungsmedium, z.B. Wasser, bildet sich eine Stoßwelle aus. Diese wird durch eine akustische Linse fokussiert, deren Brennpunkt sich nach einem Einstellvorgang im Konkrement des Patienten befindet. Dabei kann es sich z.B. um einen Nierenstein handeln.

Die Ortung des Konkrements hat dabei eine hohe Bedeutung. Je größer die Zielgenauigkeit ist, desto größer ist der therapeutische Erfolg und desto kleiner die Belastung des Patienten. Es ist bekannt, die Ortung mit Röntgengeräten vorzunehmen. Allerdings kann dabei wegen der Strahlenbelastung nicht während der gesamten Stoßwellenbehandlung die Lage des Steins überprüft werden. Es werden jeweils nur von Zeit zu Zeit Röntgenbilder aufgenommen, um die Lage des Steins zu überprüfen. Für eine kontinuierliche Überwachung der Steinlage ist man deswegen bereits dazu übergegangen, die Ortung des Konkrements mit Ultraschall durchzuführen. So z.B. ist aus der DE-PS 34 27 001 ein Ortungs-und Positionierverfahren bekannt, bei welchem mittels eines Ultraschallschwingers das Konkrement geortet wird, vorgegebene Markierungsmarken gesetzt werden und anschließend eine mechanische Korrelation der Position des Konkrements mit dem Brennpunkt des Stoßwellensystems vorgenommen wird.

Aus der DE-OS 31 19 295 ist weiterhin bekannt, die Ortung des Konkrements mit der Stoßwellenquelle selbst vorzunehmen. Im dort geschilderten System ist die Stoßwellenquelle eine Anordnung aus einer Vielzahl piezoelektrischer Wandlerelemente. Dieses Verfahren kann allerdings nur bei Stoßwellenquellen, deren Stoßwellenimpuls mit piezoelektrischen Elementen hervorgerufen ·wird, angewendet werden.

Die Erfindung geht aus von der Überlegung, daß eine Überwachung und Auswahl des Laufweges der Stoßwellenimpulse mit Hilfe der Ultraschall-Ortungssignale auch bei einem Lithotripter wünschenswert ist, der den Stoßwellenimpuls ohne piezoelektrische Elemente erzeugt.

Aufgabe der Erfindung ist es, einen Lithotripter der eingangs genannten Art so auszugestalten. daß eine fortlaufende Ultraschall-Ortung des Konkrements unabhängig von der Stoßwellenquelle möglich ist. Nach Möglichkeit soll auch gleichzeitig eine Überprüfung des Laufweges der Stoßwellenimpulse möglich sein.

Diese Aufgabe wird erfindungsgemäß dadurch gelöst, daß -in Ausbreitungsrichtung der Stoßwellenimpulse gesehen hinter oder vor der Fokussierungseinrichtung - das dreh-oder - schwenkbare Element eines mechanischen Sektorscanners angeordnet ist, dessen Drehachse senkrecht auf der Zentralachse der Fokussierungseinrichtung steht.

Man kann dies auch so ausdrücken: Es ist ein Ultraschall-Sektorscanner vorgesehen, dessen Abtaststrahl von einem Punkt ausgeht, der auf der Zentralachse liegt, und zwar entweder zwischen Fokussierungseinrichtung und Austrittsfenster am Patienten oder aber zwischen der Stoßwellenquelle und der Fokussierungseinrichtung.

Durch diese Maßnahme kann unabhängig von der Stoßwellenquelle die Steinortung im Echtzeit-Betrieb vorgenommen werden. Das Konkrement läßt sich kontinuierlich beobachten, so daß Verschiebungen, z.B. aufgrund der Atemtätigkeit, während der gesamten Behandlungsdauer verfolgt werden können.

Eine besonders vorteilhafte Ausführungsform der Erfindung zeichnet sich dadurch aus, das das Element ein pendelnder Ultraschall-Wandler oder Ultraschall-Spiegel ist, der um einen Gesamtwinkel von + 90° bis - 90° schwenkbar ist; hierbei ist eine Synchronisiereinrichtung, die den Auslösezeitpunkt jedes Stoßwellenimpulses mit der Winkelstellung des Ultraschall-Wandlers synchronisiert, vorgesehen. Mit Hilfe der Synchronisiereinrichtung wird der Stoßwellenimpuls jeweils dann ausgelöst, wenn der Ultraschall-Wandler mit seiner relativ großen Stirnfläche zur Seite zeigt und so nicht zu einer nennenswerten Abschattung des Stoßwellenimpulses führt. Lediglich die seitliche Projektionsfläche des Ultraschall-Wandlers bringt eine geringe unerwünschte Dämpfung des Stoßwellenimpulses mit sich.

Ist als Fokussierungseinrichtung beispielsweise eine akustische Sammellinse vorgesehen. so kann der Ultraschall-Sektorscanner im ersten Fokus der Linse, der der Stoßwellenquellenseite zugeordnet ist, angeordnet werden. Der Ultraschall-Sektorscanner sendet von dem ersten Fokus Ultraschall-Ortungsimpulse als Abtaststrahlen aus, die fächerförmig auf der Linsenoberfläche auftreffen. Die Linse kann dabei als bikonkave Linse konzipiert sein. Durch die Linse werden die Abtaststrahlen in einen Parallelscan umgerichtet. Der Parallelscan erfaßt dann das Gebiet, in welchem das Konkrement im Patienten liegt, und einen großen Teil der Umgebung dazu. Wird z.B. die Lage des Fokus der Stoßwellenimpulse in das Ultraschall-Bild eingeblendet, so kann eine Verschiebung des Konkrements aus dem Fokus heraus ohne Schwierigkeiten erfaßt und beobachtet werden. Dieses ist im Echtzeit-Betrieb während der gesamten Lithotripsiebehandlung des Patienten möglich.

Weitere Vorteile und Ausgestaltungen der Erfindung ergeben sich aus der Beschreibung von Ausführungsbeispielen anhand von drei Figuren. Gleiche Bauteile sind mit denselben Bezugszeichen belegt.

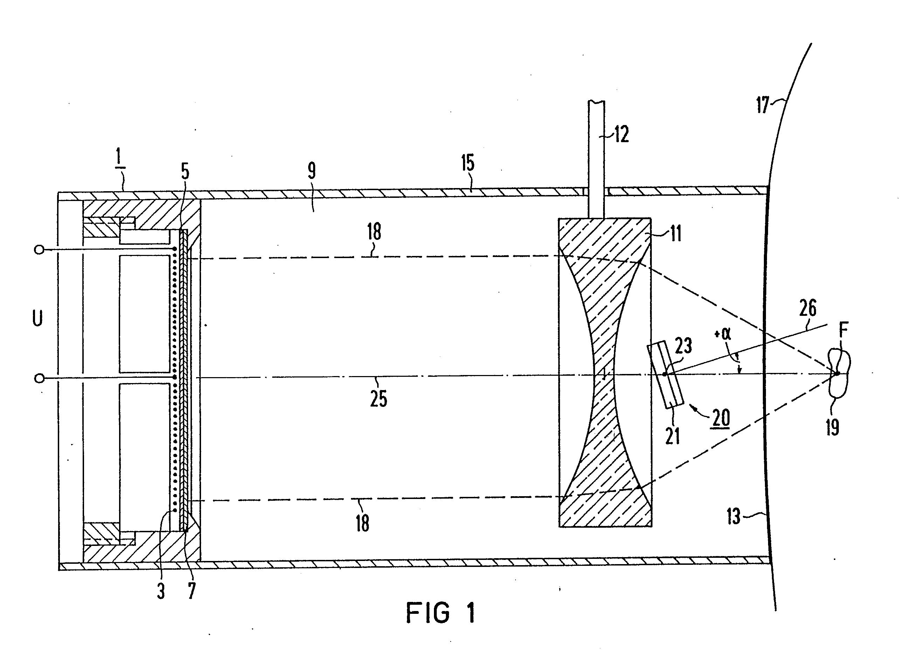

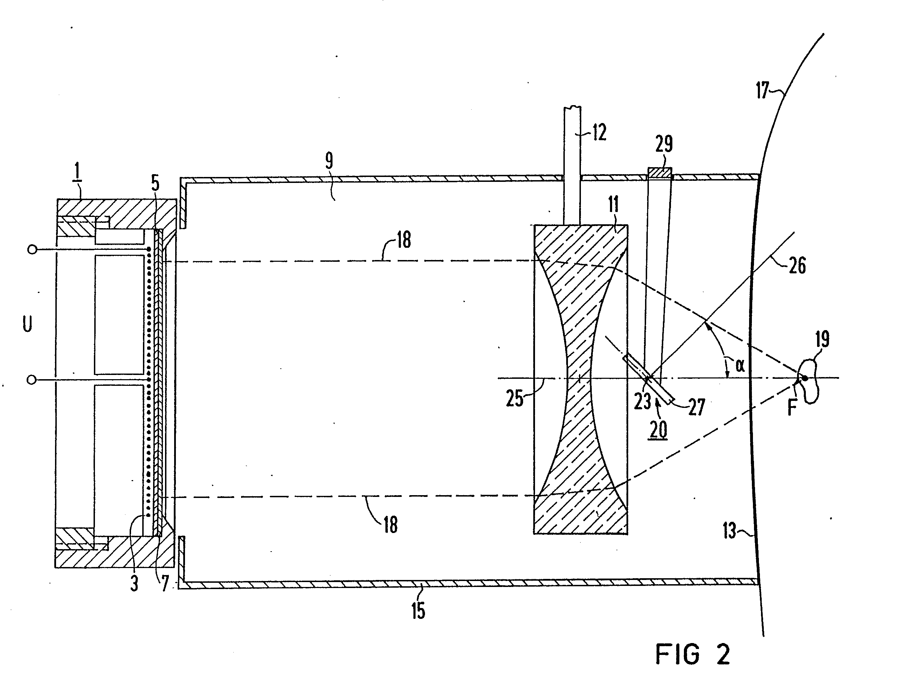

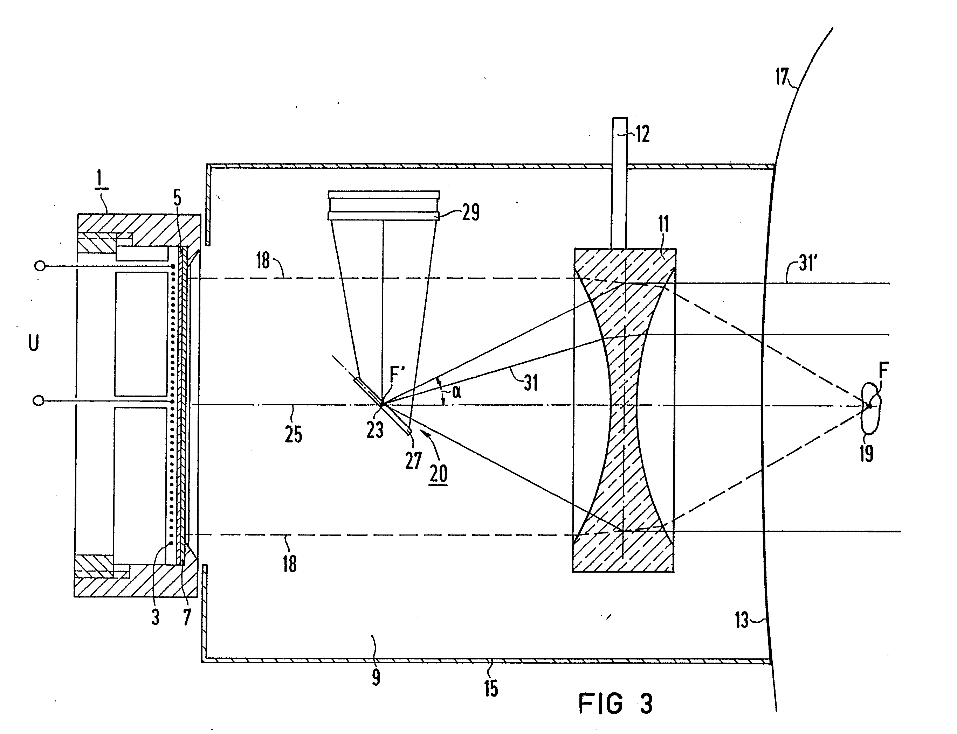

Fig. 1 zeigt einen Lithotripter mit einem Stoßwellenrohr und mit einem pendelnden Ultraschall-Wandler im Strahlengang. Fig. 2 zeigt eine Ausführung mit drehbarem Ultraschall-Spiegel und seitlich fest angeordnetem Ultraschall-Wandler. Fig. 3 zeigt einen Lithotripter mit einem Stoßwellenrohr und einem Sektorscanner im Strahlengang zwischen einer Stoßwellenquelle und einer Fokussierungseinrichtung.

In Fig. 1 ist eine Stoßwellenquelle in Form eines sog. "Stoßwellenrohrs" dargestellt. Die Stoßwellenquelle 1 umfaßt eine flache elektrische Spule 3 in Spiralkonfiguration, vor der, durch eine Isolierfolie 5 getrennt, eine Metallmembran 7 (z.B. aus Kupfer) angeordnet ist. Die Spule 3 kann mit einem Spannungsimpuls U beaufschlagt werden. An die Metallmembran 7 schließt sich eine Wasservorlaufstrecke 9 an. In der Wasservorlaufstrecke 9 befindet sich als Fokussierungseinrichtung eine Linse 11, die durch Haltemittel 12 positioniert ist. Die Wasservorlaufstrecke 9 wird stirnseitig von einer Ankoppelmembran 13 abgeschlossen. Ein zylindrisches Rohrteil 15, welches stirnseitig zum einen von der Stoßwellenquelle 1 und zum anderen von der Ankoppelmembran 13 abgeschlossen ist, bildet ein Gehäuse. Dies ist mit der Koppelflüssigkeit Wasser gefüllt.

Die Ankoppelmembran 13 ist an die Hautoberfläche eines Patienten 17 angelegt. Die Stoßwellenquelle 1 ist dabei so positioniert, daß der Fokus F der Linse 11 mit der Lage eines Konkrements 19, z.B. eines Nierensteins, zusammenfällt. Der Stoßwellenweg ist durch gestrichelte Begrenzungslinien 18 verdeutlicht.

In Stoßwellen-Ausbreitungsrichtung gesehen hinter der Linse 11 befindet sich das dreh-oder - schwenkbare Element 20, hier insbesondere der Ultraschall-Wandler 21, eines mechanischen Sek torscanners. Die Drehachse 23 des Ultraschallwandlers 21 steht senkrecht auf der Zentralachse 25 der Fokussierungseinrichtung oder Linse 11. Der Ultraschall-Wandler 21 ist ein pendelnder Wandler, dessen Abstrahl-und Empfangsrichtung 26 jeweils um einen Winkel Alpha beiderseits zur Zentralachse 25 schwenkbar ist. Der Gesamtwinkel 2 Alpha ist vorzugsweise durch die Endwerte ± 90° gegeben, beträgt also 180°. Der Sektor-Abtaststrahl geht somit von einem Punkt aus, der auf der Zentralachse 25 liegt, und zwar zwischen der Linse 11 und der Membran 13.

Der Sektorscanner weist Antriebs-und Rückmeldeglieder für den Ultraschall-Wandler 21 auf, die weitgehend außerhalb des Schallweges der Stoßwellenimpulse angeordnet sind. Die Antriebs-und Rückmeldeglieder umfassen insbesondere eine mechanische Welle, die zum rotatorischen Antrieb des Ultraschall-Wandlers 21 vorgesehen ist und elektrische Zu-und Ableitungen für den Ultraschall-Wandler 21 enthält. Diese Welle (nicht gezeigt) fällt mit der Drehachse 23 zusammen.

Selbstverständlich gehört zu dem Wandler 21 eine (nicht gezeigte) Ansteuer-und Auswerte-Elektronik, die von konventionellem Aufbau sein kann.

Es ist eine Synchronisierungseinrichtung (nicht gezeigt) vorgesehen, die den Auslösezeitpunkt jedes Stoßwellenimpulses mit der Winkelstellung Alpha des Ultraschall-Wandlers 21 synchronisiert. Die Synchronisierungseinrichtung ist vorzugsweise so ausgebildet, daß nur bei einer Winkelstellung Alpha gleich + 90° (1. Seitwärtsstellung) und Alpha gleich - 90° (2. Seitwärtsstellung) jeweils ein Stoßwellenimpuls den Wandler 21 passiert. Dadurch wird der Stoßwellenimpuls durch den Ultraschall-Wandler 21 nur geringfügig abgeschattet und in seiner Wirkung nur wenig vermindert. Außerdem wird so die Zerstörungswirkung des Stoßwellenimpulses auf den Ultraschall-Wandler 21 gering gehalten.

Der Ultraschall-Wandler 21 umfaßt beispielsweise einen Array von Ultraschall-Wandler-Elementen, deren Abstrahleinrichtung 26 durch Drehung des Arrays um die Drehachse 23 veränderlich ist, z.B. ein Annular Array. Es kann sich aber auch um ein einzelnes Übertragerelement handeln.

Alternativ hierzu kann nach Fig. 2 der mechanische Sektorscanner als dreh-oder schwenkbares Element 20 einen um die Achse 23 drehbaren Ultraschall-Spiegel 27 umfassen, der hinter der Fokussierungseinrichtung (Linse) 11 angeordnet ist und der von einem seitlich und relativ zur Spiegel-Drehachse 23 feststehend angeordneten Ultraschall-Wandler oder -Übertrager 29 beschallt wird. Der Ultraschall-Übertrager 29 ist hier im oder am Rohrteil 15 befestigt. Ultraschall-Spiegel 27 und Ultraschall-Übertrager 29 wirken hier gemeinsam wie der drehbare Ultraschall-Wandler 21 in Fig. 1 für Sendung und Empfang. Die Drehachse 23 steht hier wieder senkrecht auf der Zentralachse 25. Die Sende-und Empfangsrichtung ist wieder mit 26 bezeichnet. Auch hier geht also der Abtaststrahl von einem Punkt aus, der auf der Zentralachse 25 liegt. Ensprechend Fig. 1 ist auch hier wieder eine (nicht gezeigte) Synchronisierungseinrichtung vorgesehen.

In den Fällen nach Fig. 1 und 2 ist es angebracht, die Drehachse 23 des Ultraschall-Wandlers 21 bzw. des Ultraschall-Spiegels 27 um die Zentralachse 25 der Linse 11 um einen Winkel Beta (nicht gezeigt) drehbar auszuführen. Dadurch wird die Möglichkeit geschaffen, nicht lediglich einen ebenen Sektor abzutasten, sondern einen Kegel innerhalb des Patienten 17 zu beschallen. Auch kann dadurch eine besonders zweckmäßige Abtastebene ausgewählt werden.

Sinnvollerweise ist der Ultraschall-Wandler 21 oder Spiegel 27 dicht vor der Ankoppelmembran 13 angeordnet, die zur Ankopplung der Stoßwellenimpulse in den Körper des Patienten 17 dient, um Bildstörungen durch Mehrfachreflektionen zu minimieren. Bei einer Tiefenjustage des Stoßwellenfokus durch Abstandsänderung zwischen der Linse 11 und der Koppelmembran 13 sollte also der Spiegel 27 oder der Wandler 21 in festem Abstand zur Membran 13 gehalten werden.

Die Einrichtung nach Fig. 1 und 2 schafft die Möglichkeit, während der gesamten Lithotripsiebehandlung die Lage des Konkrements 19 zu verfolgen. Etwaige Verschiebungen aus dem Fokus F heraus können rechtzeitig beobachtet und durch Nachjustieren der Stoßwellenquelle 1 oder andere Mittel, z.B. Anwendung eines flexiblen Ankoppelsacks, korrigiert werden.

Eine weitere (nicht gezeigte) Ausführungsform verwendet zwei mechanische Sektorscannersysteme etwas außermittig, symmetrisch zur Zentralachse 25 dicht beieinander angebracht, die senkrecht aufeinanderstehende Ebenen abtasten und gleichzeitig darstellen. Es ist hier also z.B. ein zweiter Ultraschall-Wandler vorgesehen, dessen Abstrahleinrichtung senkrecht zu der Abstrahleinrichtung 26 des ersten Ultraschall-Wandlers 21 liegt. Entsprechendes gilt für die Verwendung von zwei Ultraschall-Spiegeln.

Auch bei Ausführungsform nach Fig. 3 ist die Stoßwellenquelle 1 so positioniert, daß der zweite Fokus F der Linse 11 mit der Lage des Konkrements 19 zusammenfällt. In Stoßwellen-Ausbreitungsrichtung gesehen vor der Linse 11 befindet sich hier im ersten Fokus F' als Element 20 der Ultraschall-Spiegel 27, der von dem seitlich angeordneten Ultraschall-Übertrager 29 beschallt wird. Der Ultraschall-Spiegel 27 wirkt zusammen mit dem Ultraschall-Übertrager 29 wie der dreh- oder schwenkbare Ultraschall-Wandler 21 in Fig. 1, nämlich als Sektorscanner, der sektorförmig einen Abtaststrahl 31 aussendet. Der Ultraschall-Spiegel 27 weist hier eine Drehachse 23 auf, die im ersten Fokus F' senkrecht auf der Zentralachse 25 der Fokussierungseinrichtung oder Linse 11 steht. Der schwenkbare Abtaststrahl 31 geht also vom Punkt F' aus. Mittels der Linse 11 wird patientenseitig aus dem Sektor-Scan ein Parallel-Scan, was durch den Abtaststrahl 31, 31' in Fig. 3 gezeigt ist.

Alternativ zu der Kombination aus Ultraschall-Spiegel 27 und Ultraschall-Übertrager 29 kann auch hier entsprechend Fig. 1 - was nicht gezeigt ist - ein Ultraschall-Wandler 21 (sei es ein Einzelwandler, sei es ein Array, wie z.B. ein Annular Array, aus piezoelektrischen Ultraschall-Elementen) verwendet werden. Dieser Wandler 21 wäre wie der Spiegel 27 anzuordnen und zu bewegen, um den Scan durchzuführen.

Der in Fig. 3 dargestellte Sektorscanner weist Antriebs-, Ansteuer-und Rückmeldeglieder für Spiegel 27 und den Ultraschall-Wandler 29 auf, die weitgehend außerhalb des Schallwegs der Stoßwellenimpulse angeordnet sind. Die Antriebs-und Rückmeldeglieder umfassen auch hier insbesondere eine mechanische Welle, die zum rotatorischen Antrieb des Ultraschall-Spiegels 27 (bzw. des Ultraschall-Wandlers 21) vorgesehen ist und ggf. elektrische Leitungen für den Ultraschall-Wandler 21 enthält. Diese Welle (nicht gezeigt) fällt mit der Drehachse 23 zusammen.

Es ist wiederum eine Synchronisierungseinrichtung (nicht gezeigt) vorgesehen, die den Auslösezeitpunkt jedes Stoßwellenimpulses mit der Winkelstellung Alpha des Elements 27 (oder 21) synchronisiert. Die Synchronisierungseinrichtung ist wieder vorzugsweise so ausgebildet, daß nur bei einer Winkelstellung Alpha von ± 90° jeweils ein Stoßwellenimpuls am Spiegel 27 oder Wandler 21 vorbeiläuft. In diesen beiden Stellungen (Seitwärtsstellungen) wird der Stoßwellenimpuls durch den Ultraschall-Spiegel 27 bzw. den Ultraschall-Wandler 21 nur geringfügig abgeschattet.

Es ist auch hier angebracht, die Drehachse 23 des Ultraschall-Spiegels 27 (bzw. des Ultraschall-Wandlers 21) um die Zentralachse 25 der Linse 11 um einen Winkel Beta drehbar auszuführen. Dadurch wird wieder die Möglichkeit geschaffen, den Patienten 17 nicht lediglich in einem ebenen Parallelscan abzutasten, sondern einen Zylinder innerhalb des Patienten 17 zu beschallen oder auch eine besonders zweckmäßige Abtastebene auszuwählen.

Vorteil des in Fig. 3 dargestellten Lithotripters ist es, daß zusammen mit der Verdrehung der Abtastebene der gesamte Schallzugang für den Stoßwellenimpuls untersucht werden kann. Mit anderen Worten, der gesamte Weg des Stoßwellenimpulses kann überwacht, die Ankopplung kann überprüft und das Vorhandensein von Schallhindernissen wie Luft oder Knochen kann festgestellt werden. Damit kann eine Optimierung der Ankopplung bzw. der Einschallrichtung vorgenommen werden. Zu diesem Zweck ist es sinnvoll, den Weg des Stoßwellenimpulses in das B-Bild des Ultraschall-Gerätes einzublenden. Mehrfachechos, die möglicherweise innerhalb der Linse 11 hervorgerufen werden und zu Doppelbildern führen könnten, können durch Vergütung der Linse 11 unterdrückt werden.

Wenn die Drehachse 23 vor oder hinter dem ersten Fokus F' angeordnet wird, kann (statt des Parallel-Scans) ein Divergent-oder Convergent-Scan erzeugt werden.

Eine weitere, auf Fig. 3 aufbauende (nicht gezeigte) Ausführungsform verwendet zwei mechanische Sektorscansysteme etwas außermittig, symmetrisch zur Zentralachse 25 dicht beieinander angebracht, die senkrecht aufeinanderstehende Ebenen abtasten und gleichzeitig darstellen.

## Ansprüche

1. Lithotripter zur Zertrümmerung eines Konkrementes im menschlichen Körper mit einem Stoßwellenrohr (15), das an seinem einen Ende mit einem Stoßwellenimpulse aussendenden Stoßwellenerzeuger (3, 7) und an seinem anderen Ende mit einer Ankoppelmembran (13) abgeschlossen ist und mit einer Fokussierungseinrichtung (11) im Stoßwellenrohr (15), **daurch gekennzeichnet,** daß -in Ausbreitungsrichtung der Stoßwellenimpulse gesehen hinter (Fig. 1 und 2) oder vor (Fig. 3) der Fokussierungseinrichtung (11) - das dreh-oder schwenkbare Element (20; 21; 27) eines mechanischen Sektorscanners angeordnet ist, dessen Drehachse (23) senkrecht auf der Zentralachse (25) der Fokussierungseinrichtung (11) steht.

2. Lithotripter nach Anspruch 1 , **dadurch gekennzeichnet,** daß der Sektorscanner einen pendelnden Ultraschall-Wandler (21) oder Ultraschall-Spiegel (27) umfaßt, der um einen Winkel (Alpha) ± 90° schwenkbar ist.

3. Lithotripter nach Anspruch 1 oder 2 , **dadurch gekennzeichnet,** daß der Sektorscanner Antriebsund Rückmeldeglieder für das dreh-oder -schwenkbare Element (21; 27) aufweist, die weitgehend außerhalb des Schallweges (20) der Stoßwellenimpulse angeordnet sind.

4. Lithotripter nach Anspruch 3 , **dadurch gekennzeichnet,** daß die Antriebs-und Rückmeldeglieder eine mechanische Welle umfassen, die zum rotatorischen Antrieb des Elements (21; 27) vorgesehen ist und bevorzugt auch elektrische Leitungen für das Element (21; 27) enthält.

5. Lithotripter nach einem der Ansprüche 1 bis 5 , **gekennzeichnet durch** eine Synchronisierungseinrichtung, die den Auslösezeitpunkt jedes Stoßwellenimpulses mit der Winkelstellung (Alpha) des Elements (21; 27) synchronisiert.

6. Lithotripter nach Anspruch 5 , **dadurch gekennzeichnet,** daß die Synchronisierungseinrichßung so ausgebildet ist, daß nur bei einer Winkelstellung Alpha = + 90° und/oder Alpha = - 90° jeweils ein Stoßwellenimpuls am Element (21; 27) vorbeiläuft.

7. Lithotripter nach einem der Ansprüche 1 bis 6 , **dadurch gekennzeichnet,** daß ·das Element (21) einen Einzelwandler oder aber ein Array von Ultraschall-Wandlerelementen umfaßt, deren Abstrahleinrichtung (26) durch Drehung des Arrays veränderlich ist.

8. Lithotripter nach einem der Ansprüche 1 bis 6 , **dadurch gekennzeichnet,** daß der mechanische Sektorscanner als das Element einen drehbaren Ultraschall-Spiegel (27) umfaßt, der hinter bzw. vor der Fokussierungseinrichtung (11) angeordnet ist, und daß ein Ultraschall-Wandler (29) vorgesehen ist, der seitlich feststehend angeordnet, zur Beschallung des Ultraschall-Spiegels (27) sowie zum Empfang vorgesehen ist (Fig. 2 bzw. 3).

9. Lithotripter nach einem der Ansprüche 1 bis 8 , **dadurch gekennzeichnet,** daß die Drehachse (23) des Elements (21; 27) um die Zentralachse (25) der Fokussierungseinrichtung (11) um einen vorgegebenen Winkel drehbar ist.

10. Lithotripter nach einem der Ansprüche 1 bis 9 , **dadurch gekennzeichnet,** daß das Element 1 (21; 27) dicht vor einer Ankoppelmembran (13) angeordnet ist, die zur Einkopplung der Stoßwellenimpulse in den Körper eines Patienten (17) dient.

11. Lithotripter nach einem der Ansprüche 1 bis 10 , **dadurch gekennzeichnet,** daß ein zweites dreh-oder schwenkbares Element vorgesehen ist, dessen Abstrahlrichtung senkrecht zu der Abstrahlrichtung (26) des ersten Elements (21; 27) liegt.

12. Lithotripter nach einem der Ansprüche 1 bis 11 , **dadurch gekennzeichnet,** daß der Abtaststrahl (31) des Ultraschall-Scanners von einem Punkt (F') ausgeht, der auf der Zentralachse (25) zwischen der Stoßwellenquelle (1) und der Fokussierungseinrichtung (11) liegt (Fig. 3).

13. Lithotripter nach einem der Ansprüche 9 bis 12 , **dadurch gekennzeichnet,** daß der Ultraschall-Sektorscanner um die Zentralachse (25) um den vorgegebenen Winkel von mindestens 90° drehbar ist.

87 P 3020 E

FIG 1

FIG 2

87 P 3020 E

0 278 303

FIG 3

0 278 303

87 P 3020 E

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| P,X | EP-A-0 221 592 (PHILIPS GmbH) <br> * Seite 3, Zeilen 27-33; Seite 4, Zeile 21 - Seite 5, Zeile 17; Figur 1 * <br> ----- | 1,3,4,7,9 | A 61 B 17/22 |
| | | | **RECHERCHIERTE SACHGEBIETE (Int. Cl.4)** <br><br> A 61 B <br> G 10 K |

Der vorliegende Recherchenbericht wurde für alle Patentansprüche erstellt

| Recherchenort | Abschlußdatum der Recherche | Prüfer |
|---|---|---|
| DEN HAAG | 21-04-1988 | NEILL M.C. |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus andern Gründen angeführtes Dokument
 
& : Mitglied der gleichen Patentfamilie, übereinstimmendes
    Dokument

EPO FORM 1503 03.82 (P0403)